# EUROPEAN PATENT APPLICATION

(11) **EP 4 492 119 A1**
(43) Date of publication of application: **15.01.2025**
(21) Application number: 22930923.2
(22) Date of filing: 11.03.2022
(51) Int. Cl.: G02B 21/36, C12M 1/00, G01B 9/02, G01N 15/00, G01N 21/17

(54) **IMAGING DEVICE AND IMAGING METHOD**

(71) Applicant: Nikon Corporation, Tokyo 140-8601 (JP)
(72) Inventor: MORIYAMA Masaki, Tokyo 108-6290 (JP); ISHIZAWA Naoya, Tokyo 108-6290 (JP); KOBAYASHI Ryo, Tokyo 108-6290 (JP); TAKUBO Makiko, Tokyo 108-6290 (JP); HAYASHI Seri, Tokyo 108-6290 (JP); TANAKA Shuhei, Tokyo 108-6290 (JP); MURAKAMI Masataka, Tokyo 108-6290 (JP); HOSHINO Tetsuro, Tokyo 108-6290 (JP); IWASA Akio, Tokyo 108-6290 (JP); MIMURA Masafumi, Tokyo 108-6290 (JP); FUKUTAKE Naoki, Tokyo 108-6290 (JP); NOZAKI Kiyoshi, Tokyo 108-6290 (JP)
(74) Representative: Hoffmann Eitle
(86) International application number: PCT/JP2022/010954
(87) International publication number: WO 2023/170942

(57) **Abstract**

An imaging device (10) includes a substrate (1), a first optical system (2), and an imaging part (3). A subject (110) is disposed on the substrate (1). The first optical system (2) irradiates the subject (110) with first emitted light (L1). The imaging part (3) images an interference image between first reflected light (L2) and second reflected light (L3). The first reflected light (L2) is the first emitted light (L1) reflected at a first interface (F1) conforming to an outer surface of the subject (110). The second reflected light (L3) is the first emitted light (L1) reflected at a second interface (F2) between the subject (110) and the substrate (1).

## Description

### Technical Field

The present invention relates to an imaging device and an imaging method.

### Background Art

In cell biology research or the like, imaging devices are used to observe cell shapes using interference images obtained by light reflected from cell surfaces (for example, see Non-Patent Document 1). In the imaging device, it is required to accurately understand a three-dimensional shape of a subject.

### Citation List

### Non-Patent Document

Non-Patent Document 1: T. Yamauchi et. al., "Low coherent quantitative phase microscope for nanometer scale measurement of living cells morphology," Optics Express 2008, Vol.16, No. 16, 12227-12238.

### Summary of Invention

An aspect of the present invention is an imaging device including: a substrate on which a subject is disposed; a first optical system configured to irradiate the subject with first emitted light; and an imaging part configured to image an interference image between first reflected light and second reflect light, the first reflected light being the first emitted light reflected at a first interface conforming to an outer surface of the subject and the second reflected light being the first emitted light reflected at a second interface between the subject and the substrate.

An aspect of the present invention is an imaging method including: disposing a subject on a substrate; irradiating the subject with first emitted light; and imaging an interference image between first reflected light and second reflected light, the first reflected light being the first emitted light reflected at a first interface conforming to an outer surface of the subject and the second reflected light being the first emitted light reflected at a second interface between the subject and the substrate.

### Brief Description of Drawings

[FIG.1] FIG. 1 is a schematic diagram showing an imaging device of an embodiment.
[FIG.2] FIG. 2 is a schematic diagram showing a structure of a gas supply part.
[FIG.3] FIG. 3 is a schematic diagram showing the gas supply part and a substrate.
[FIG.4] FIG. 4 is an enlarged schematic diagram showing the substrate and a subject.
[FIG. 5] FIG. 5 is a further enlarged schematic diagram of the substrate and the subject.
[FIG.6] FIG. 6 is an example of an interference image.
[FIG.7] FIG. 7 is a view showing brightness of the interference image along line A-A' in FIG. 6.
[FIG. 8] FIG. 8 is an example of the interference image including a transmitted image.
[FIG.9] FIG. 9 is an enlarged view of FIG. 8.
[FIG. 10] FIG. 10 is a view for describing an imaging method of an embodiment.
[FIG. 11] FIG. 11 is a view for describing the imaging method of the embodiment.
[FIG. 12] FIG. 12 is an example of the interference image.
[FIG. 13] FIG. 13 is a view showing chronological change of the brightness along line A-A' in FIG. 12.
[FIG. 14] FIG. 14 is a view showing chronological change of brightness at a center of interference fringes along line B-B' of FIG. 13.
[FIG. 15] FIG. 15 is a brightness profile along line C-C' in FIG. 13. Description of Embodiments

Hereinafter, embodiments of the present invention will be described with reference to the accompanying drawings in detail where necessary. Further, in the drawings, the same or corresponding parts are designated by the same or corresponding signs, and overlapping descriptions will be omitted. Further, the dimensional ratios in each drawing may be exaggerated for the purpose of description and may not necessarily correspond to the actual dimensional ratios.

### [Imaging device]

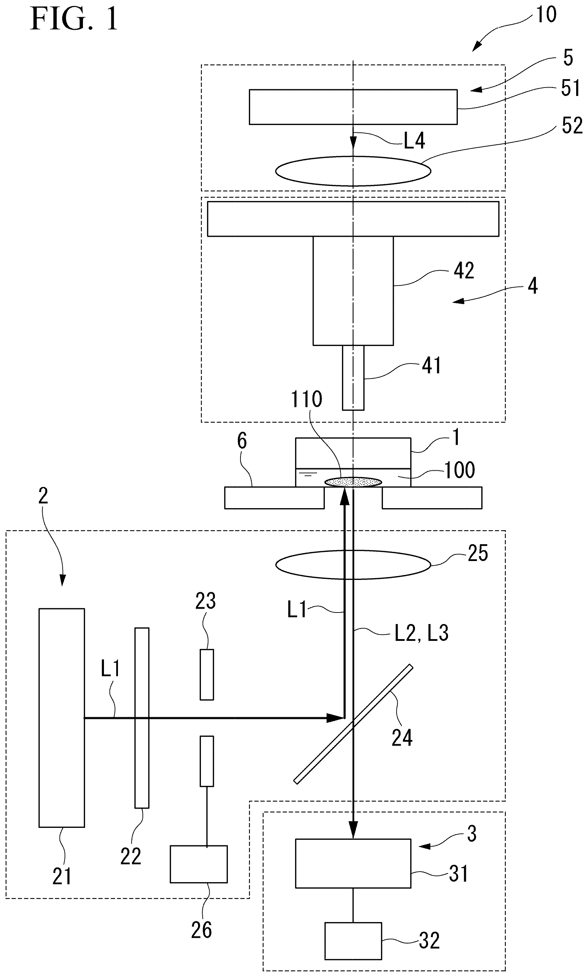

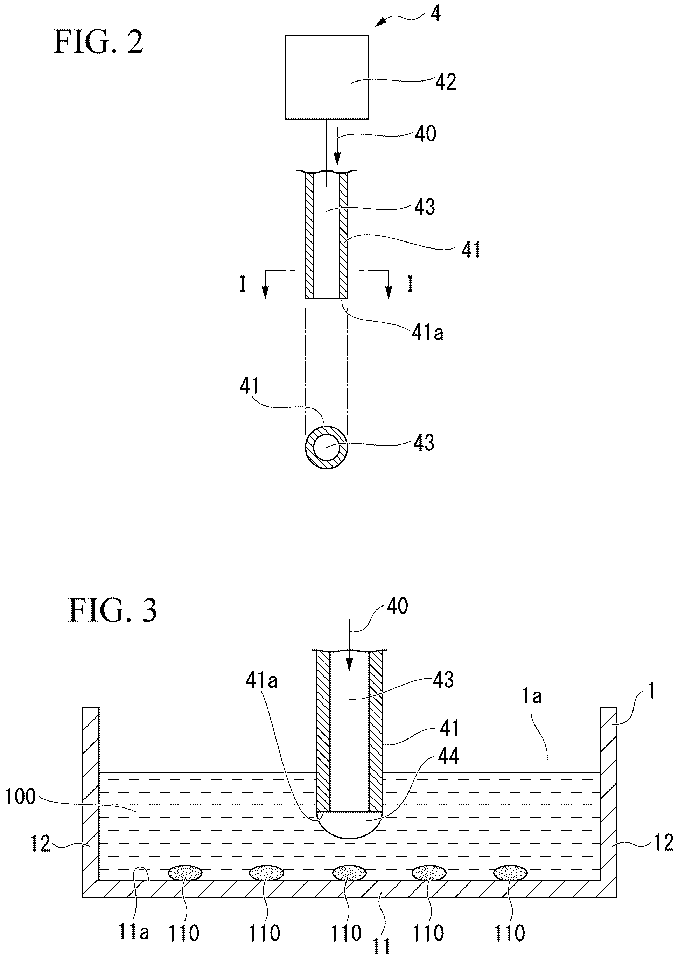

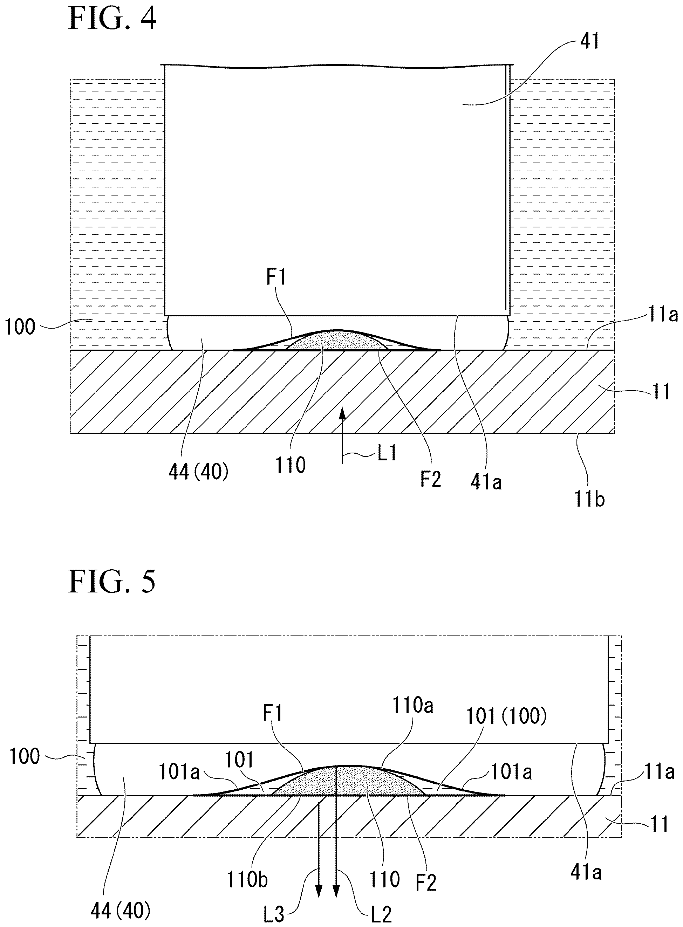

FIG. 1 is a schematic diagram of an imaging device 10 of an embodiment. FIG. 2 is a schematic diagram showing a structure of a gas supply part 4. FIG. 2 shows a cross-sectional view along a central axis of a hollow member 41 and a cross-sectional view along line I-I perpendicualr to the central axis. FIG. 3 is a schematic diagram showing the hollow member 41 and a substrate 1. FIG. 4 is an enlarged schematic diagram of a bottom plate 11 and a subject 110 of the substrate 1. FIG. 5 is a further enlarged schematic diagram of the bottom plate 11 and the subject 110. "When seen in a plan view" refers to as when seen in a direction perpendicular to an upper surface 11a of the bottom plate 11.

As shown in FIG. 1, the imaging device 10 of the embodiment includes the substrate 1, a first optical system 2, an imaging part 3, the gas supply part (material supply part) 4, a second optical system 5, and a stage 6.

As shown in FIG. 3, the substrate 1 includes the bottom plate 11, and side plates 12. The side plates 12 stand up from a circumferential edge of the bottom plate 11. The substrate 1 can store liquid 100 in an internal space 1a defined by the bottom plate 11 and the side plates 12. The upper surface 11a of the bottom plate 11 is formed flat. The substrate 1 can hold the liquid 100 and the subject 110.

While the substrate 1 shown in FIG. 3 includes the bottom plate 11 and the side plates 12, a structure of the substrate is not limited thereto. For example, a flat substrate constituted by only the bottom plate 11 may be used if it can hold droplets and the subject.

The bottom plate 11 allows first emitted light L1 from the first optical system 2 to pass therethrough (see FIG. 4). The bottom plate 11 may have a refractive index difference of 0.1 or more with the subject 110. By setting the refractive index of the bottom plate 11 in this range, reflectivity at an interface between the bottom plate 11 and the subject 110 (a second interface F2) can be increased. Either the refractive index of the bottom plate 11 or the refractive index of the subject 110 may be higher. The refractive index of the bottom plate 11 may be for example, 1.45 to 2.1. Measurement methods for refractive index include JIS K7142:2014.

As the material of the substrate 1, for example, polystyrene, polycarbonate, polymethyl methacrylate (PMMA), polyethylene, polypropylene, poly-4-methylpentene-1, glass, and the like, are exemplified. The refractive index of the polystyrene is about 1.5. The refractive index of polycarbonate is about 1.6. The refractive index of PMMAis about 1.5. The refractive index of polyethylene and polypropylene is about 1.5. The refractive index of poly-4-methylpentene-1 is about 1.46. The refractive index of glass is, for example, 1.4 to 2.1.

The upper surface 11a of the substrate 1 may be subjected to surface treatment to enhance cell adhesion. As the surface treatment, physical hydrophilic treatment such as plasma treatment or the like, or application of cell adhesion substances such as gelatin, fibronectin, collagen, extracellular matrix, or the like, is exemplified.

The shape of the substrate 1 is not particularly limited. The substrate 1 may be, for example, petri dishes; well plates such as 6-well, 12-well, 24-well, 48-well, 96-well, or the like, plates; fluidic devices; glass slides, or the like.

As shown in FIG. 1, the first optical system 2 includes a first light source 21, a band pass filter 22, an aperture diaphragm 23, a half mirror 24, an objective lens 25, and an aperture controller 26.

As the first light source 21, for example, a mercury lamp, a halogen lamp, a laser light source, or the like, may be used.

A wavelength of the first emitted light L1 from the first light source 21 may include a near infrared region. Accordingly, even if there is a material inside the subject 110 that causes absorption or scattering in the visible light region, the absorption or scattering of light in the subject 110 can be suppressed. Accordingly, it is possible to reduce noise that affects the interference image.

The wavelength of the near infrared region is, for example, 650 nm to 1.4 µm.

The wavelength of the first emitted light L1 may include 650 nm or more. Accordingly, for example, when the subject 110 is a cell, it is possible to obtain first reflected light L2 without being affected by an intercellular substance (organelles or the like) that causes absorption or scattering in the visible light region. Accordingly, it is possible to obtain the interference image with less noise.

The wavelength of the first emitted light L1 may be, for example, 1.7 µm or less.

The wavelength width of the first emitted light L1 may be 50 nm or less. By setting the wavelength width in this range, the coherence length can be increased. Since the interference between the first reflected light and the second reflected light occurs in the range of the coherence length, the coherence length is essentially an upper limit to the measurable thickness of the subject 110. Since the coherence length can be increased by setting the wavelength width to this range, a good interference image can be obtained even when the thickness of the subject 110 is large.

The wavelength width of the first emitted light L1 may be, for example, 0.1 nm or more.

The wavelength width of the first emitted light L1 may be 1/10 or less of the wavelength of the first emitted light L1. Since the coherence length is determined by the wavelength and the wavelength width, it is preferable to appropriately determine the wavelength width for the wavelength. Since the wavelength width of the first emitted light L1 is equal to or less than 1/10 of the wavelength, a good interference image can be obtained even when the thickness of the subject 110 is large.

The wavelength width of the first emitted light L1 may be 1/1000 or more of the wavelength.

The first emitted light L1 may include a plurality of wavelengths (i.e., a plurality of wavelength components) with a wavelength difference of 20 nm or less. Accordingly, the effective central wavelength becomes longer. If the thickness of the subject 110 falls within one interference fringe, information about the height of the outer surface of the subject 110 can be obtained from the light and shade of the interference image. Since information on the height of the outer surface of the subject 110 can be obtained without the need for complex analysis of interference fringes, the shape of the subject 110 can be understood easily and accurately.

The first emitted light L1 may include a plurality of wavelengths with a wavelength difference of 1 nm or more.

The band pass filter 22 selectively transmits light having predetermined characteristics (for example, a wavelength, a wavelength width, and the like).

The half mirror 24 reflects the first emitted light L1 from the first light source 21 toward the substrate 1. The half mirror 24 transmits the first reflected light L2 and a second reflected light L3 toward the imaging part 3.

The aperture diaphragm 23 can adjust a numerical aperture of illumination by the first light source 21 by adjusting a diameter of a luminous flux of the first emitted light L1.

The aperture controller 26 controls a diameter of the aperture diaphragm 23. The aperture controller 26 can reduce, for example, a numerical aperture of illumination by reducing the diameter of the aperture diaphragm 23. Accordingly, it is possible to obtain an interference image with clear interference fringes. Meanwhile, since the illumination light illuminates the subject 110 through the objective lens 25, when the numerical aperture of the objective lens 25 is smaller compared to the numerical aperture of the illumination, the numerical aperture of the objective lens 25 becomes a numerical aperture of final illumination. For this reason, the numerical aperture of the illumination controlled by the aperture diaphragm 23 may be smaller than the numerical aperture of the objective lens 25. The numerical aperture of the objective lens 25 may be 0.7 or less.

In addition, an imaging numerical aperture is determined solely by the numerical aperture of the objective lens 25. The imaging numerical aperture may be 0.7 or less.

The first optical system 2 can irradiate the subject 110 with the first emitted light L1 from the first light source 21 from below the substrate 1 through the bottom plate 11 (see FIG. 4).

The imaging part 3 includes a detector 31, and an information processor 32.

The detector 31 includes, for example, an image sensor such as a CCD, a CMOS, or the like. The detector 31 images an image of the subject 110. The image sensor may be an area sensor, may be a linear sensor, or may be a point sensor. A detection signal corresponding to the detected light is appropriately A/D-converted by an A/D converter or the like and output to the information processor 32.

The information processor 32 processes the information on the basis of the detection signal from the detector 31.

As shown in FIG. 2, the gas supply part 4 includes the hollow member 41, and a controller 42.

The hollow member 41 is formed, for example, in a circular tube shape (or a cylindrical shape). The hollow member 41 has a flow path 43 through which a gas 40 flows. The hollow member 41 can release the gas 40 from a tip portion 41a by delivering the gas 40 into the flow path 43. When the tip portion 41a is in the liquid 100, air bubbles 44 are formed in the tip portion 41a (see FIG. 3). The gas 40 is an example of "the material." The gas 40 is, for example, air. The gas 40 may have a refractive index smaller than that of the subject 110 by 0.1 or more.

The gas supply part 4 can maintain the air bubbles 44 by closing a flow path in the gas supply part 4 or the flow path 43 such that the gas 40 does not flow back into the hollow member 41. The air bubbles 44 may be maintained by adjusting the amount of the gas 40 drawn in or supplied from the gas supply part 4, thereby adjusting at least one of the pressure and volume of the air bubbles 44.

The hollow member 41 may be configured to be movable in a direction perpendicular to the central axis of the hollow member 41 (horizontal direction) by a drive unit such as a motor (not shown) or the like.

The controller 42 may include, for example, a pump or the like configured to supply a gas 40. The controller 42 can control a supply amount of the gas 40. For this reason, the controller 42 can adjust a volume of the air bubbles 44 (see FIG. 3).

The controller 42 may include, for example, a pressure sensor or the like configured to a pressure of the gas 40 (a pressure in the flow path 43). The controller 42 can control the pressure of the gas 40 by controlling the supply amount of the gas 40 using the pump configured to supply the gas 40. For this reason, the controller 42 can adjust the pressure of the air bubbles 44 (see FIG. 3).

As shown in FIG. 1, the second optical system 5 includes a second light source 51, and a condensing lens 52.

The second optical system 5 can irradiate the subject 110 with second emitted light L4 from above.

The stage 6 can have the substrate 1 placed on its upper surface. The stage 6 can be moved in a direction perpendicular to the central axis of the hollow member 41 (horizontal direction) by a drive unit (not shown) such as a motor or the like.

### [Imaging method] (First embodiment)

As shown in FIG. 3, the subject 110 and the liquid 100 are accommodated in the internal space 1a of the substrate 1. The liquid 100 is, for example, water. The refractive index of the water is about 1.33. The liquid 100 may be a liquid culture medium for culturing the subject 110. The refractive index of the liquid culture medium is also about 1.33. The subject 110 is disposed on the substrate 1 (specifically, on the bottom plate 11).

The subject 110 is disposed in the liquid 100. That is, the subject 110 is immersed in the liquid 100. The subject 110 is, for example, a cell. The cell may be an animal cell, a plant cell, an insect cell, a germ, yeast, or the like. The cell may be an adherent cell, or may be a suspension cell. Even in the case of the suspension cell, imaging is possible if the substrate 1 is left stationary and the cell is allowed to sink until it contacts the bottom plate 11. The refractive index of the cell is for example, about 1.35 to 1.60.

The subject 110 is not particularly limited, but it is preferable that when light passes through the subject 110, the refractive index difference at the interface is large, because this results in high reflectivity and a clear interference image. An object that has a small absorption rate but has a refractive index distribution is called a phase object. In the phase object, if the refractive index difference at the interface is large, the reflectivity becomes high and clear interference fringes can be obtained.

The hollow member 41 of the gas supply part 4 is disposed such that the tip portion 41a is disposed in the liquid 100. Apart of the hollow member 41 including the tip portion 41a is immersed in the liquid 100.

The gas 40 is supplied to the flow path 43 of the hollow member 41 by the controller 42 (see FIG. 2), and the air bubbles 44 are formed on the tip portion 41a. The refractive index of the gas 40 is different from the refractive index of the liquid 100.

As shown in FIG. 4 and FIG. 5, the subject 110 comes into surface contact with the upper surface 11a of the bottom plate 11. Specifically, as shown in FIG. 5, a lower surface 110b of the subject 110 comes into contact with the upper surface 11a of the bottom plate 11 over the entire region. In FIG. 5, the subject 110 has an arch shape formed by a straight line forming the lower surface 110b and a circular arc forming an upper surface 110a. The upper surface 110a of the subject 110 is an outer surface of the subject 110.

The interface between the bottom plate 11 and the subject 110 is the second interface F2.

When the hollow member 41 is brought close to the subject 110, the air bubbles 44 cover the subject 110. The air bubbles 44 surround the subject 110 when seen in a plan view. Here, some of the liquid 100 in contact with the subject 110 is pushed outward by the air bubbles 44.

Some of the liquid 100 is remained in the portion including the circumferential edge portion of the subject 110. The remained liquid 100 is referred to as residual liquid 101. The residual liquid 101 forms a layer that gradually thins outward in the radial direction of the subject 110.

The residual liquid 101 may also be present as a thin layer in a center portion of the upper surface 110a of the subject 110 when seen in a plan view. In this case, the residual liquid 101 is present throughout the entire circular region centered on the subject 110 when seen in a plan view.

The residual liquid 101 may remain only in the annular portion of the subject 110 including the circumferential edge portion. In this case, the residual liquid 101 becomes annular that surrounds the center portion of the subject 110 when seen in a plan view.

When the residual liquid 101 is present throughout the entire circular region centered on the subject 110, the interface between the residual liquid 101 and the gas 40 is a first interface F1. When the residual liquid 101 is present in an annular shape, the first interface F1 is formed by the annular interface between the residual liquid 101 and the gas 40 and the circular interface between the center portion of the subject 110 and the gas 40.

At least a part of the first interface F1 is formed between the residual liquid 101 and the gas 40 (a gas having a refractive index different from that of the residual liquid 101).

Since the residual liquid 101 is flowable and has surface tension, an upper surface 101a of the residual liquid 101 becomes a smooth shape that conforms to an upper surface (outer surface) 110a of the subject 110. For this reason, the first interface F1 has a smooth shape that conforms to the upper surface (outer surface) 110a of the subject 110.

In a state the first interface F1 is formed by the air bubbles 44, the pressure of the gas 40 in the air bubbles 44 can be adjusted by adjusting the supply amount of the gas 40 using the controller 42, as necessary. While the subject 110 (cell) may be deformed when the pressure of the gas 40 is too high, the controller 42 can accurately understand a three-dimensional shape of the subject 110 by controlling the supply amount of the gas 40 such that deformation of the subject 110 does not become large.

In a state the first interface F1 is formed by the air bubbles 44, the stage 6 (see FIG. 1) may be moved in a direction perpendicular to the central axis of the hollow member 41 (a horizontal direction), if necessary. Accordingly, since the position of the tip portion 41a with respect to the subject 110 can be adjusted, a shape of the first interface F1 can be arranged.

With the first interface F1 formed by the air bubbles 44, the hollow member 41 may be moved in a direction perpendicular to the central axis of the hollow member 41 (a horizontal direction), if necessary. Accordingly, since the position of the tip portion 41a with respect to the subject 110 can be adjusted, a shape of the first interface F1 can be arranged.

As shown in FIG. 1, the first light source 21 emits the first emitted light L1. The first emitted light L1 enters the band pass filter 22. The band pass filter 22 selectively transmits light with predetermined characteristics. The first emitted light L1 passing through the band pass filter 22 reaches the half mirror 24, after the diameter of the luminous flux is adjusted at the aperture diaphragm 23. The first emitted light L1 is reflected by the half mirror 24, travels upward, passes through the objective lens 25, and enters the bottom plate 11 of the substrate 1.

As shown in FIG. 4, the first emitted light L1 enters the bottom plate 11 from a lower surface 11b, passes upward through the bottom plate 11, and reaches the second interface F2.

As shown in FIG. 5, the bottom plate 11 (the refractive index is, for example, about 1.5) and the subject 110 (the refractive index is, for example, about 1.35) have different refractive indices. For this reason, some of the first emitted light L1 is reflected by the second interface F2. The reflected light is referred to as "the second reflected light L3."

As shown in FIG. 1, the second reflected light L3 travels downward, passes through the objective lens 25 and the half mirror 24, and reaches the detector 31 of the imaging part 3.

As shown in FIG. 5, the first emitted light L1 (see FIG. 4) that is not reflected at the second interface F2 travels upward and reaches the first interface F1.

The residual liquid 101 (the refractive index is, for example, about 1.33) or the subject 110 (the refractive index is, for example, about 1.35) and the gas 40 (the refractive index is, for example, 1) have different refractive indices. For this reason, some of the first emitted light L 1 is reflected by the first interface F1. The reflected light is referred to as "the first reflected light L2."

As shown in FIG. 1, the first reflected light L2 travels downward and passes through the subject 110 or the residual liquid 101 again. The first reflected light L2 further passes through the bottom plate 11 and travels downward, and passes through the objective lens 25 and the half mirror 24 to reach the detector 31 of the imaging part 3.

The first reflected light L2 and the second reflected light L3 enter the detector 31. For this reason, in the imaging part 3, an interference image is imaged based on the phase difference between the first reflected light L2 and the second reflected light L3. The detector 31 sends data of the obtained interference image to the information processor 32. In the information processor 32, a three-dimensional image of the subject 110 can be acquired on the basis of the interference image.

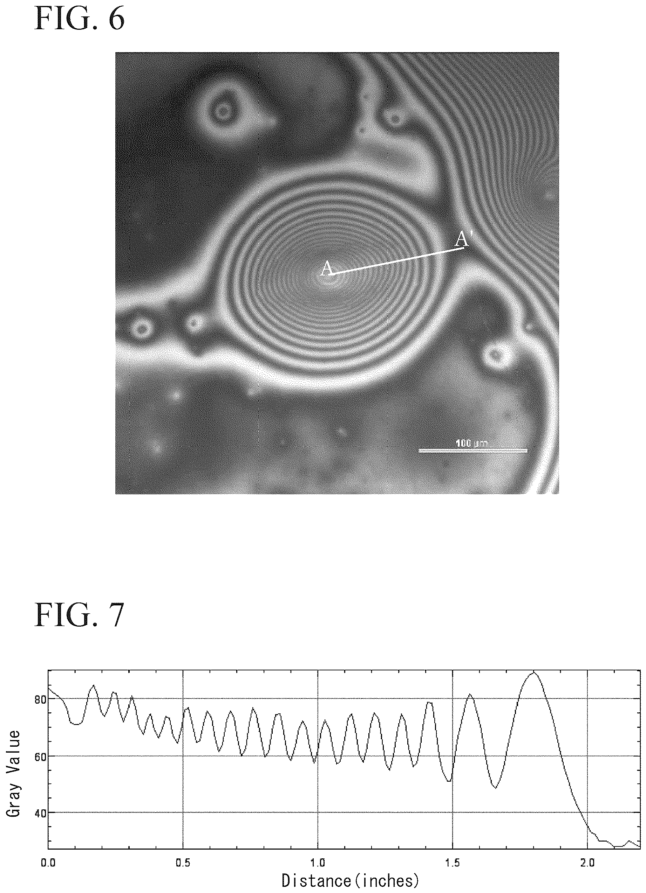

FIG. 6 is an example of the interference image. FIG. 7 is a view showing brightness of the interference image along line A-A' in FIG. 6. A shape (height) of the first interface F1 can be calculated on the basis of the brightness shown in FIG. 7. In this way, it is possible to understand a three-dimensional shape of the subject 110 on the basis of the interference image.

As shown in FIG. 5, since the residual liquid 101 (the liquid between the first interface F1 and the second interface F2) is present near the circumferential edge portion of the subject 110, in the interference image (see FIG. 6), the interference fringes are observed in a range wider than the subject 110.

In order to understand the shape of the subject 110 when seen in a plan view, a heat may be generated by irradiation with the first emitted light L1 and at least some of the residual liquid 101 may be evaporated. Accordingly, the first interface F1 is shaped to conform to the outer surface of the subject 110, resulting in the interference image that conforms to the subject 110. Accordingly, it is possible to accurately understand the shape of the subject 110.

The imaging device 10 of the first embodiment includes the imaging part 3 configured to image an interference image between the first reflected light L2 at the first interface F1 and the second reflected light L3 at the second interface F2. The first interface F1 is an interface between the residual liquid 101 (or the subject 110) and the gas 40. Since the first interface F1 is the interface between the two materials having a large refractive index difference, reflectivity is high. For example, a good interference image with clear interference fringes can be obtained. Accordingly, it is possible to accurately understand a three-dimensional shape of the subject 110.

When the subject 110 is the cell, a subcellular organelles or the like is present in the cell. The subcellular organelles or the like has a refractive index higher than that of cell nucleus, cytoplasm, or the like (for example, mitochondria: about 1.4, lysosome: 1.6). For this reason, due to differences in the refractive index between the subcellular organelles and cell nuclei, irregular reflections occur within the cells, which can become noise components of the interference fringes. Meanwhile, the first interface F1 has a very large refractive index difference between the cell and the material (gas). For this reason, the reflectivity of the first interface F1 becomes high, and the reflected light from the first interface F1 becomes dominant, so that the influence of reflected light from subcellular organelles or the like can be ignored. Accordingly, good interference fringes can be obtained. Accordingly, it is possible to accurately understand a three-dimensional shape of the subject 110.

On the other hand, when the subject 110 is placed in water, since the refractive index difference between the water and the subject 110 is small, the reflectivity of the first interface (the interface between the water and the subject 110) tends to be low. For this reason, there is a possibility that the reflected light at the first interface will become weaker and the interference image may become unclear.

In the imaging method of the first embodiment, the first interface F1 is formed with high reflectivity and little distortion of a plane wave, resulting in high-quality first reflected light L2. For this reason, it is possible to obtain a clear interference image by the first reflected light L2 and the second reflected light L3. Accordingly, it is possible to accurately understand a three-dimensional shape of the subject 110.

### [Imaging method]

### (Second embodiment)

As shown in FIG. 1, in an imaging method of the second embodiment, like the imaging method of the first embodiment, the substrate 1 and the subject 110 are irradiated with the first emitted light L1 from the first light source 21. The first reflected light L2 and the second reflected light L3 enter the imaging part 3.

In the imaging method of the second embodiment, the second optical system 5 is used. The second light source 51 of the second optical system 5 emits the second emitted light L4. The second emitted light L4 is transmitted downward through the subject 110 and the bottom plate 11. The second emitted light L4 passes through the objective lens 25 and the half mirror 24 and enters the detector 31 of the imaging part 3.

In the imaging part 3, in addition to the interference image, the transmitted image of the subject 110 is obtained by the second emitted light L4. The transmitted image overlaps the interference image.

In the imaging method of the second embodiment, an interference image containing information from the transmitted image is obtained. For this reason, the interference image that includes information about external appearance of the subject 110 can be obtained.

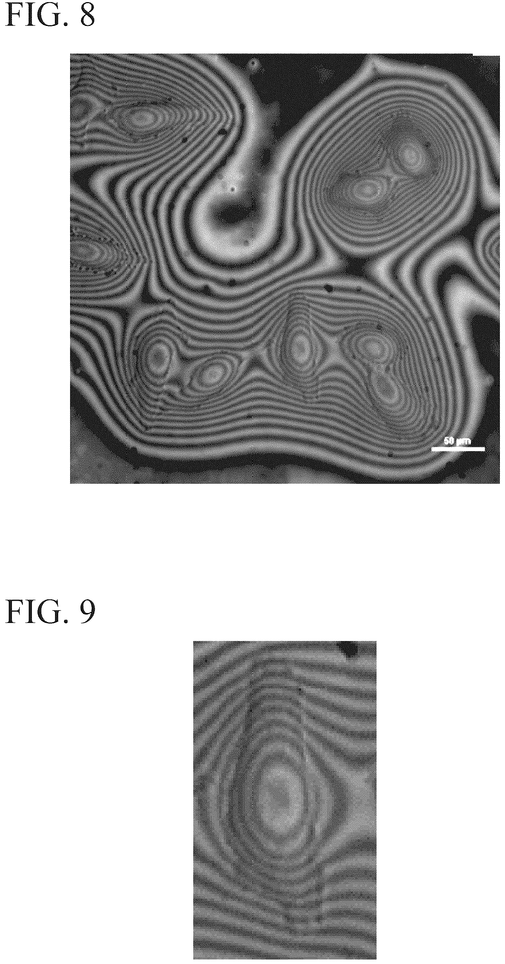

FIG. 8 is an example of the interference image including the transmitted image. FIG. 9 is an enlarged view of FIG. 8. As shown in FIG. 8 and FIG. 9, the transmitted image can be seen inside the interference image which has interference fringes.

### [Imaging method]

### (Third embodiment)

As shown in FIG. 4 and FIG. 5, in an imaging method of a third embodiment, the hollow member 41 is disposed such that the air bubbles 44 cover the subject 110.

The controller 42 increases or decreases the supply amount of the gas 40 to the hollow member 41. Accordingly, the pressure of the gas 40 is changed. Here, the shape of the subject 110 understood from the interference image acquired by the imaging part 3 is also changed. In the information processor 32, viscoelasticity information or surface tension of the subject 110 can be acquired from the pressure change of the gas 40, the shape change of the subject 110, and a time required for the pressure change and the shape change.

Under certain conditions, since the pressure (surface stress) applied to the subject 110 can be regarded as the pressure of the gas 40, there is no need to consider the tension of the first interface F1, but there is a case in which it is necessary to consider the tension specific to the first interface F1.

Since the surface stress applied to the subject 110 by the first interface F1 can be calculated from an eigenvalue of the tension and the interface shape (a deformation amount of the interface) of the first interface F1, it can be calculated on the basis of the shape of the first interface F1 obtained from the interference image. For example, when the first interface F1 is the interface between the air and the liquid, it is known that the stress (plasma pressure) occurs due to surface tension and the radius of curvature of the interface. The plasma pressure can be obtained from the shape of the first interface F1 (a radius of curvature of the interface) understood from the surface tension (eigenvalue) and the interference image. In the information processor 32 of the imaging part 3, it is possible to acquire the surface stress applied to the subject 110 from the shape information and physical properties of the interface.

For example, in a state in which the first interface F1 is formed by the air bubbles 44, the first interference image when the pressure of the gas 40 is set as a first pressure and the second interference image when the pressure of the gas 40 is set as a second pressure (a pressure different from the first pressure) may be acquired.

In the information processor 32 of the imaging part 3, it is possible to obtain viscoelasticity information of the subject 110 on the basis of the first interference image and the second interference image. Here, the time change from the first pressure to the second pressure and the shape change of the subject may be used to analyze viscoelasticity information.

### [Imaging method]

### (Fourth embodiment)

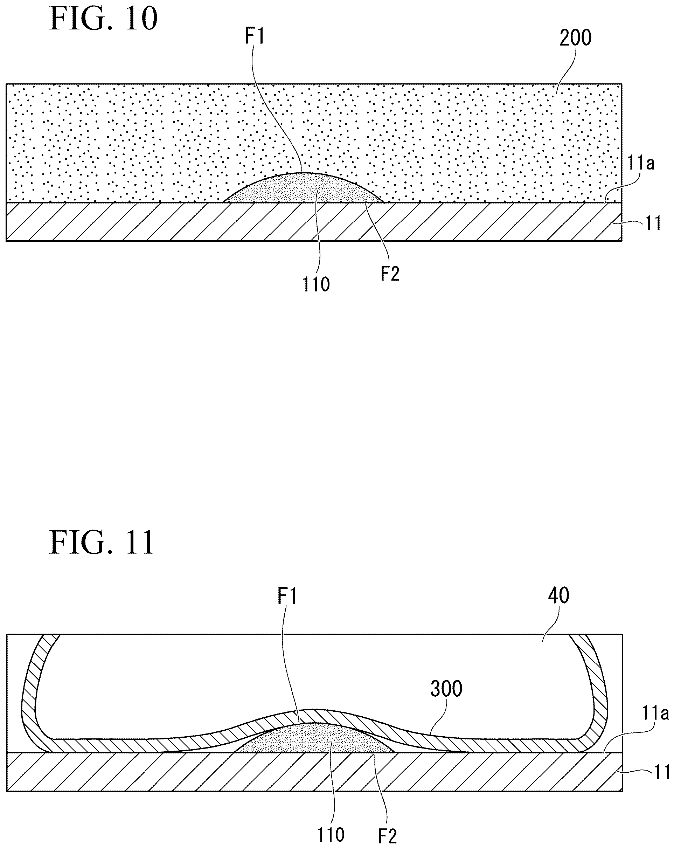

FIG. 10 is a view for describing an imaging method of a fourth embodiment.

A material in contact with the subject may have a refractive index greater than that of the subject. For example, as shown in FIG. 10, liquid 200, which contains subject 110, has a higher refractive index than the subject 110. The liquid 200 may have, for example, a refractive index greater than that of the subject 110 by 0.1 or more. The refractive index of the liquid 200 is, for example, 1.45 or more. As the liquid 200, a glycerin aqueous solution, a sugar solution, or the like, is exemplified. The first interface F1 is an interface between the liquid 200 and the subject 110.

Even in the embodiment, it is possible to accurately understand the three-dimensional shape of the subject 110 using the interference image between the first reflected light L2 at the first interface F1 and the second reflected light L3 at the second interface F2.

### [Imaging method]

### (Fifth embodiment)

FIG. 11 is a view for describing an imaging method of a fifth embodiment.

A material in contact with the subject 110 may be a thin film member 300. The thin film member 300 has a refractive index different from that of the subject 110 or properties of reflecting light. The thin film member 300 has, for example, a refractive index greater than that of the subject 110 by 0.1 or more. The refractive index of the thin film member 300 is, for example, 1.45 or more. The thin film member 300 is formed of, for example, a resin. The thin film member 300 overlaps the upper surface 110a of the subject 110. The first interface F1 is an interface between the thin film member 300 and the subject 110.

The thin film member 300 may be formed into a bag or balloon shape that is inflated by introducing a gas through the hollow member 41. The thin film member 300 can be held by the tip portion 41a of the hollow member 41 (see FIG. 3). The position and shape of the thin film member 300 can be adjusted by adjusting a position of the tip portion 41a or an internal pressure of the hollow member 41. For this reason, like the gas 40 (see FIG. 4 and FIG. 5), it is possible to control the pressure applied to the subject 110. Further, the position and shape of the thin film member 300 may be adjusted by controlling at least one of the pressure and volume of the gas supplied or drawn through the hollow member 41.

Even in the embodiment, it is possible to accurately understand the three-dimensional shape of the subject 110 using the interference image between the first reflected light L2 at the first interface F1 and the second reflected light L3 at the second interface F2.

### [Imaging method]

### (Sixth embodiment)

To understand a three-dimensional image of the subject 110, it is desirable to set the position where the first interface F1 reaches the substrate 1 (specifically, the upper surface 1 1a of the bottom plate 11) as the reference position for the height of the subject 110. However, if an attempt is made to expose the upper surface 11a of the bottom plate 11 by increasing the pressure of the gas 40 in the air bubbles 44 (see FIG. 5), the subject 110 may be deformed by the pressure of the gas 40. For this reason, it is effective to understand the shape of the subject 110 when no pressure from the gas 40 is applied (original shape: shape before deformation) by the following method. Further, the position where the first interface F1 reaches the substrate 1 may be a position of a part of the surface in close proximity where a small amount of water exists between the first interface F1 and the substrate 1 in a state in which the first interface F1 and the substrate 1 are brought sufficiently close to each other.

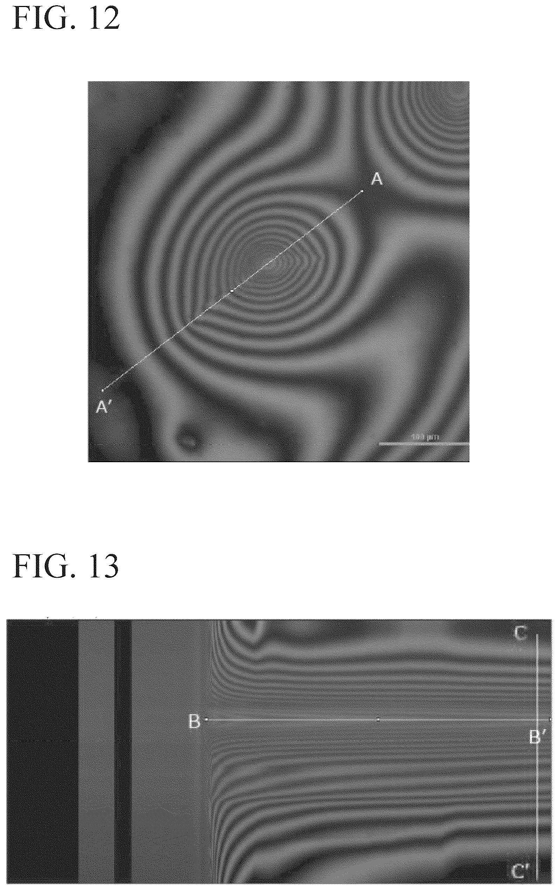

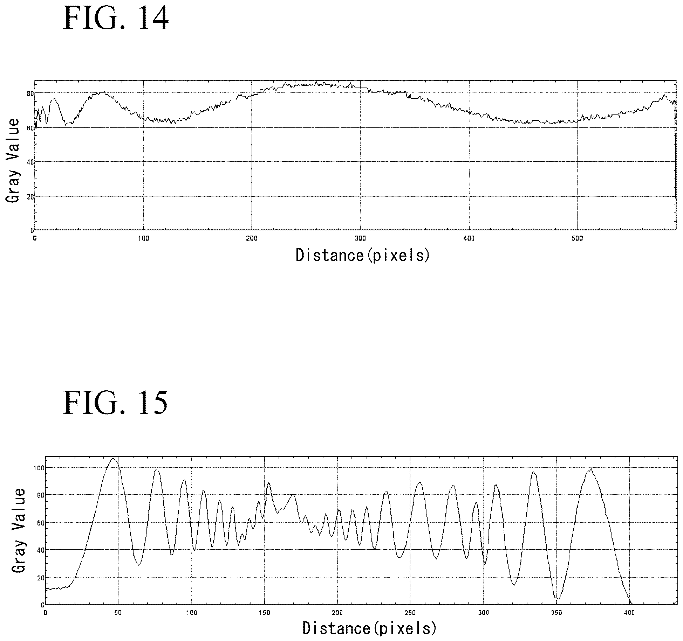

FIG. 12 is an example of an interference image. FIG. 13 is a view showing a chronological change of brightness along line A-A' in FIG. 12. In FIG. 13, a lateral axis indicates a time. A vertical axis indicates brightness along line A-A'. FIG. 14 is a view showing a chronological change of the brightness at the center of the interference fringes. FIG. 15 is a brightness profile along line C-C' in FIG. 13.

As shown in FIG. 13, when the pressure of the gas 40 is applied to the subject 110 by pressing the air bubbles 44 against the subject 110 (see FIG. 5), the interference fringes appear (point B). The interference fringes of the interference image change over time until the final point (point B').

As shown in FIG. 14, the brightness at the center of the interference fringes increases or decreases. Since the changes in the interference fringes appeared herein indicate a deformation process of the subject 110 and the brightness profile at the final point (B' point) shown in FIG. 15 indicates the shape of the subject 110 at the final point, it is possible to predict and understand the original shape of the subject 110 before the pressure of the gas 40 was applied, on the basis of the information of the deformation process and the final shape.

Meanwhile, by the following method, the first interface F1 can be controlled to reach the substrate 1 while keeping the pressure of the gas 40 small. It is possible to expose the upper surface 11a of the bottom plate 11 by adjusting affinity between the first interface F1 and the substrate 1 without increasing the pressure of the gas 40. For example, in a case in which the liquid 100 is a liquid culture medium, when the liquid culture medium is replaced with a phosphate buffer with high surface tension, the affinity of the gas 40 with the substrate 1 is increased, and the first interface F1 easily approaches or reaches the upper surface 1 1a of the bottom plate 11. Similarly, the affinity of the gas 40 with the substrate 1 is increased by making the upper surface 11a of the bottom plate 11 water repellent, and the first interface F1 easily approaches or reaches the upper surface 1 1a of the bottom plate 11.

Hereinabove, although one embodiment of the present invention has been described in detail with reference to the drawings, the specific configuration is not limited to the above, and various design changes can be made within the scope of the present invention without departing from the scope of the present invention.

In the imaging device 10, the first optical system 2 irradiates the subject 110 with light from below to obtain an interference image. The second optical system 5 configured to obtain a transmitted image irradiates the subject 110 with light from above. The configuration of the imaging device is not limited to the example. The first optical system may irradiate the subject with light from below. The second optical system may irradiate the subject with light from above.

### Reference Signs List

1 Substrate
2 First optical system
3 Imaging part
4 Gas supply part (material supply part)
5 Second optical system
10 Imaging device
21 First light source
23 Aperture diaphragm
26 Aperture controller
31 Detector
32 Information processor
41 Hollow member
43 Flow path
44 Air bubbles
51 Second light source
F1 First interface
F2 Second interface

## Claims

1. An imaging device comprising:
a substrate on which a subject is disposed;
a first optical system configured to irradiate the subject with first emitted light; and
an imaging part configured to image an interference image between first reflected light and second reflect light, the first reflected light being the first emitted light reflected at a first interface conforming to an outer surface of the subject and the second reflected light being the first emitted light reflected at a second interface between the subject and the substrate.

2. The imaging device according to claim 1, wherein the substrate has a refractive index difference of 0.1 or more with the subject.

3. The imaging device according to claim 1 or 2, wherein the substrate is able to hold liquid and the subject disposed in the liquid.

4. The imaging device according to claim 3, wherein at least a part of the first interface is formed between the liquid and a material having a refractive index different from that of the liquid, and
a material supply part configured to supply the material is further provided such that the first interface has a shape conforming to the outer surface of the subject.

5. The imaging device according to claim 4, wherein the material has a refractive index smaller than that of the subject by 0.1 or less.

6. The imaging device according to claim 4 or 5, wherein the material is a gas, and
the material supply part is a gas supply part.

7. The imaging device according to claim 6, wherein the gas supply part includes a hollow member having a tip portion disposed in the liquid, and forms the first interface by air bubbles formed and maintained on the tip portion in the liquid using the gas supplied through a flow path in the hollow member.

8. The imaging device according to any one of claims 1 to 7, wherein a wavelength of the first emitted light includes a near infrared region.

9. The imaging device according to claim 8, wherein the wavelength of the first emitted light includes 650 nm or more.

10. The imaging device according to any one of claims 1 to 9, wherein a wavelength width of the first emitted light is 50 nm or less.

11. The imaging device according to any one of claims 1 to 10, wherein the wavelength width of the first emitted light is 1/10 or less of the wavelength of the first emitted light.

12. The imaging device according to any one of claims 1 to 11, wherein the first optical system includes an aperture diaphragm, an aperture controller configured to control a diameter of the aperture diaphragm, and an objective lens,
the aperture controller adjusts a diameter of the aperture diaphragm such that the illumination numerical aperture is equal to or smaller than an imaging numerical aperture, and
the imaging numerical aperture is equal to or smaller than 0.7.

13. The imaging device according to any one of claims 1 to 12, wherein the first emitted light radiated from the first optical system includes a plurality of wavelength components having a wavelength difference of 20 nm or less.

14. The imaging device according to any one of claims 1 to 13, further comprising a second optical system configured to irradiate the subject with second light,
wherein the imaging part also captures the transmitted image using the second light that passes through the subject.

15. The imaging device according to any one of claims 1 to 14, wherein the imaging part includes a detector configured to image the interference image, and an information processor configured to acquire a three-dimensional image of the subject on the basis of the interference image.

16. The imaging device according to any one of claims 4 to 7, wherein the imaging part includes:
a detector configured to image the interference image; and
an information processor configured to acquire viscoelasticity information of the subject from a change in pressure of the material, a shape change of the subject calculated from the interference image, and a time required for the change in pressure and the shape change of the subject.

17. The imaging device according to claim 4, wherein the material has a refractive index greater than that of the subject.

18. The imaging device according to claim 17, wherein the material is liquid containing the subject.

19. The imaging device according to claim 17, wherein the material is a thin film member.

20. The imaging device according to any one of claims 1 to 19, wherein the subject is a phase object.

21. An imaging method comprising:
disposing a subject on a substrate;
irradiating the subject with first emitted light; and
imaging an interference image between first reflected light and second reflected light, the first reflected light being the first emitted light reflected at a first interface conforming to an outer surface of the subject and the second reflected light being the first emitted light reflected at a second interface between the subject and the substrate.

22. The imaging method according to claim 21, wherein the subject is disposed in liquid, and
at least a part of the first interface is an interface between the subject and a gas in contact with the subject.

23. The imaging method according to claim 22, wherein the gas is supplied such that the first interface has a shape conforming to an outer surface of the subject.

24. The imaging method according to claim 22 or 23, wherein the first interface is formed to conform to the subject by evaporating at least some of the liquid between the first interface and the second interface.

25. The imaging method according to claim 22 or 23, wherein the first interface is formed to conform to the subject by adjusting affinity between the gas or the liquid and the substrate.

26. The imaging method according to any one of claims 21 to 25, wherein a three-dimensional image of the subject is acquired from the interference image by using a point where the first interface has reached the substrate as a reference position of a height of the subject.

27. The imaging method according to any one of claims 22 to 26, wherein a tip portion of a hollow member having a flow path is disposed in the liquid, and
in a state in which the gas is supplied to the flow path and the first interface is formed by air bubbles formed and maintained at the tip portion in the liquid, at least one of the supply amount of the gas and the position of the tip portion is adjusted.

28. The imaging method according to any one of claims 22 to 27, wherein a first interference image when a pressure of the gas is set as a first pressure, and a second interference image when the pressure of the gas is set as a second pressure different from the first pressure, are acquired.

29. The imaging method according to claim 28, wherein viscoelasticity information of the subject is acquired on the basis of the first interference image, the second interference image, the change in pressure of the gas, the shape change of the subject calculated from the first interference image and the second interference image, and the time required for the change in pressure and the shape change of the subject.
